(19) Europäisches Patentamt
European Patent Office
Office européen des brevets

(11) EP 4 431 086 A1

(12) # EUROPEAN PATENT APPLICATION

(43) Date of publication:
**18.09.2024 Bulletin 2024/38**

(21) Application number: **23162409.9**

(22) Date of filing: **16.03.2023**

(51) International Patent Classification (IPC):
***A61K 9/20*** *(2006.01)* ***A61K 31/4015*** *(2006.01)*

(52) Cooperative Patent Classification (CPC):
**A61K 31/4015; A61K 9/2018; A61K 9/2095**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Adalvo Limited**
**San Gwann, SGN 3000 (MT)**

(72) Inventors:
- **Strusi, Orazio Luca**
  **San Gwann (MT)**
- **Samuk, Zerrin Ozge**
  **Basaksehir/Istanbul (TR)**
- **Yilmaz, Koray**
  **Kapakli/Tekirdag (TR)**

(74) Representative: **Slavík, Jiri**
**Artistu 3183/14**
**Praha - Horni Pocernice**
**193 00 Prague (CZ)**

(54) **PHARMACEUTICAL COMPOSITION COMPRISING (2S)-2-[(4R)-2-OXO-4-PROPYLTETRAHYDRO-1H-PYRROL-1-YL]BUTANAMIDE**

(57) The present invention relates to a pharmaceutical composition comprising (2S)-2-[(4R)-2-oxo-4-propyltetrahydro-1H-pyrrol-1-yl]butanamide or its pharmaceutically acceptable salts, esters, solvates, polymorphs, enantiomers or mixtures thereof, which is free of a cyclodextrin agent and free of a binding agent and to a process of the preparation thereof.

EP 4 431 086 A1

## Description

### Technical Field

The present invention relates to a pharmaceutical composition comprising (2S)-2-[(4R)-2-oxo-4-propyltetrahydro-1H-pyrrol-1-yl]butanamide or its pharmaceutically acceptable salts, esters, solvates, polymorphs, enantiomers or mixtures thereof with one or more pharmaceutically acceptable excipients, and a process of the preparation thereof.

### Background Art

Compound (2S)-2-[(4R)-2-oxo-4-propyltetrahydro-1H-pyrrol-1-yl]butanamide of formula I,

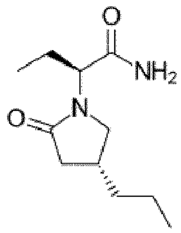

(I)

also known as Brivaracetam, exhibits antiepileptic properties and is therefore effective in the treatment of epilepsy. The compound was described for the first time in WO0162726 and WO0164637. The pharmaceutical composition comprising said active ingredient was already registered under the brand names Briviact and Nubriveo among others. According to WO0162726, Brivaracetam in a free base form has been observed in 2 solid state forms which have been characterized therein. In the pharmaceutical composition of the present invention any of these forms can be used. As reported in the European Public Assessment report for Briviact, a partial conversion of the two forms occurs in the pharmaceutical composition. Pharmaceutical compositions comprising Brivaracetam and a cyclodextrin agent are disclosed in WO2010094535 and WO2010086315. However, these compositions known from the prior art, that are using a cyclodextrin agent as a binding agent are not preferred. Cyclodextrins are expensive excipients in comparison with more commonly used binding agents. Also, the cyclodextrin amount that can be incorporated in pharmaceutical formulations is restricted for various reasons such as production capabilities, cost, formulation bulk and toxicological considerations. Moreover, bioavailability of drugs in cyclodextrin formulations can be rather limited, resulting in less-than-optimal drug delivery. Furthermore, the use of cyclodextrins may cause digestive problems such as diarrhoea and as such, are not recommended to be use in children less than 2 years old. Therefore, there remains a need to develop a simple, reproducible, a cost-effective and feasible manufacturing process with an improved flowability and processability for pharmaceutical composition of Brivaracetam, while at the same time ensuring that the desired properties such as dissolution, stability, bioavailability are maintained and are comparable with respect to the reference drug product Briviact. It is therefore the object of this invention to solve problems of the prior art and to provide a pharmaceutical composition comprising (2S)-2-[(4R)-2-oxo-4-propyltetrahydro-1H-pyrrol-1-yl]butanamide that addresses the drawbacks identified in the prior art compositions.

### Disclosure of the Invention

The present inventors have surprisingly found that a pharmaceutical composition comprising (2S)-2-[(4R)-2-oxo-4-propyltetrahydro-1H-pyrrol-1-yl]butanamide or its pharmaceutically acceptable salts, esters, solvates, polymorphs, enantiomers or mixtures thereof, which is free of a cyclodextrin agent and free of a binding agent, according to the present invention, provides for a reproducible, cost-effective and feasible manufacturing process, coupled with simpler manufacture process at industrial scale and with a similar and comparable dissolution profile to the reference drug product Briviact. This is surprising, as the composition does not replace the cyclodextrin agent (used as a binding agent) with any other binding agent and is surprisingly able to achieve better results (flowability and processability) in comparison to a formulation that has replaced the cyclodextrin agent (used as a binding agent) with another binding agent, while at the same time it beneficially remains comparable with the reference drug product Briviact (comprising cyclodextrin agent, as a binding agent).

The present invention therefore provides a pharmaceutical composition comprising (2S)-2-[(4R)-2-oxo-4-propyltetrahydro-1H-pyrrol-1-yl]butanamide or its pharmaceutically acceptable salts, esters, solvates, polymorphs, enantiomers or mixtures thereof, which is free of a cyclodextrin agent and free of a binding agent. In a preferred embodiment the pharmaceutical composition is in a form of a tablet for oral administration. Preferably the tablets are immediate release tablets and even more preferably they are immediate release film-coated tablets. Preferably, the pharmaceutical composition is further comprising excipients selected from a diluent, a disintegrant, a lubricant and/or a mixture thereof. Further preferably, the excipients are intra-granular excipients and/or extra-granular excipients or a mixture thereof. The intra-granular excipients are preferably selected from the diluent, the disintegrant, and optionally the lubricant or at least part of the lubricant. The extra-granular excipient is preferably the lubricant or at least part of the lubricant.

In another preferred embodiment, the invention provides a pharmaceutical composition comprising (2S)-2-[(4R)-2-oxo-4-propyltetrahydro-1H-pyrrol-1-yl]butanamide or its pharmaceutically acceptable salts, esters, solvates, polymorphs, enantiomers or mixtures thereof, which is free of a cyclodextrin agent and free of a binding agent further comprises:

a) a diluent which is lactose monohydrate and lactose anhydrous
b) a disintegrant which is croscarmellose sodium
c) a lubricant which is magnesium stearate, and
d) optionally a coating.

In another preferred embodiment, the invention provides a pharmaceutical composition comprising (2S)-2-[(4R)-2-oxo-4-propyltetrahydro-1H-pyrrol-1-yl]butanamide or its pharmaceutically acceptable salts, esters, solvates, polymorphs, enantiomers or mixtures thereof, which is free of a cyclodextrin agent and free of a binding agent, and further comprises:

a) around 75% wt. of a diluent or a mixture of diluents;
b) around 6% wt. of a disintegrant
c) around 1% wt. of a lubricant, and
d) optionally a coating layer of around 2% wt.

In another preferred embodiment, the invention provides a pharmaceutical composition comprising (2S)-2-[(4R)-2-oxo-4-propyltetrahydro-1H-pyrrol-1-yl]butanamide or its pharmaceutically acceptable salts, esters, solvates, polymorphs, enantiomers or mixtures thereof, which is free of a cyclodextrin agent and free of a binding agent, and further comprises:

a) around 75% wt. of a diluent which is lactose monohydrate and lactose anhydrous
b) around 6% wt. of a disintegrant which is croscarmellose sodium
c) around 1% wt. of a lubricant which is magnesium stearate, and
d) optionally a coating layer of around 2% wt.

In another preferred embodiment, the invention provides a pharmaceutical composition comprising around 18% wt. of (2S)-2-[(4R)-2-oxo-4-propyltetrahydro-1H-pyrrol-1-yl]butanamide or an equivalent amount of its pharmaceutically acceptable salts, esters, solvates, polymorphs, enantiomers or mixtures thereof, which is free of a cyclodextrin agent and free of a binding agent, and further comprises

a) around 75% wt. of a diluent which is lactose monohydrate and lactose anhydrous
b) around 6% wt. of a disintegrant which is croscarmellose sodium
c) around 1% wt. of a lubricant which is magnesium stearate, and
d) optionally a coating layer of around 2% wt.

The pharmaceutical composition according to the present invention comprises from about 15% wt. to about 20% wt. of (2S)-2-[(4R)-2-oxo-4-propyltetrahydro-1H-pyrrol-1-yl]butanamide or an equivalent amount of its pharmaceutically acceptable salts, esters, solvates, polymorphs, enantiomers or mixtures thereof. Preferably, the pharmaceutical composition comprises from about 18% wt. of (2S)-2-[(4R)-2-oxo-4-propyltetrahydro-1H-pyrrol-1-yl]butanamide or an equivalent amount of its pharmaceutically acceptable salts, esters, solvates, polymorphs, enantiomers or mixtures thereof. Even more preferably, the pharmaceutical composition comprises (2S)-2-[(4R)-2-oxo-4-propyltetrahydro-1H-pyrrol-1-yl]butanamide of formula I (Brivaracetam).

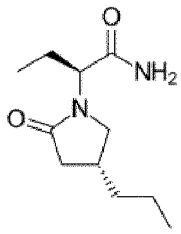

(I)

Preferably, the pharmaceutical composition according to the present invention has dissolution rate of at least 85% of the composition being dissolved at minute 10 of dissolution test, at conditions of pH 6.4 phosphate buffer with 900 ml volume, pedal apparatus with 50 rpm.

Preferably, the pharmaceutical composition according to the present invention has dissolution rate of at least 85% of the composition being dissolved at minute 15 of dissolution test, at conditions of pH 6.4 phosphate buffer with 900 ml volume, pedal apparatus with 50 rpm.

In another preferred embodiment, the invention provides for a pharmaceutical composition pharmaceutical according to the invention, wherein the particle size distribution of (2S)-2-[(4R)-2-oxo-4-propyltetrahydro-1H-pyrrol-1-yl] butanamide or its pharmaceutically acceptable salts, esters, solvates, polymorphs, enantiomers or mixtures thereof is that more than d(0.9) is from 10 μm to 100 μm, preferably, d(0.9) is from 35 μm to 60 μm, and even more preferably, d(0.9) is around 55 μm.

The invention further provides a process for the preparation of the pharmaceutical composition according to the present invention. The process is preferably a dry granulation process. The dry granulation process preferably comprises a step of roller compactor of the intra-granular excipients and a step of compression with the extra-granular excipients. The process is preferably comprising the step(s) of sifting and mixing, compaction of the intra-granular excipients, followed by sifting and mixing and compression with the extra-granular excipients, optionally followed by a coating process.

In another preferred embodiment, the process for the preparation of the pharmaceutical composition comprises the steps of:

1) Sifting and mixing: Sifting Brivaracetam, croscarmellose sodium, lactose anhydrous, lactose monohydrate by sieving 0.6 mm and mixing it for 10 minutes;
2) Sifting and mixing: Adding ½ portion of magnesium stearate by sieving 0.25 mm to the mixture from step 1 and mixing it for 3 minutes;
3) Compaction: Dry granulation of the powder obtained in step 2 and sieving;
4) Mixing: After compaction, mixing of the obtained mixture for 5 minutes;
5) Sifting and mixing: Sieving the remaining portion of magnesium stearate by 0.25 mm and adding it to the mixture from step 4 and mixing it for 3 minutes.
6) Compression: Performing tablet compression;
7) Coating: Optionally, performing a coating process.

The term "active ingredient" or "active pharmaceutical ingredient" or "Brivaracetam", used herein refers to (2S)-2-[(4R)-2-oxo-4-propyltetrahydro-1H-pyrrol-1-yl]butanamide, in a broad sense including not only Brivaracetam compound per se, but also its pharmaceutically acceptable salts, solvates, hydrates, enantiomers, derivatives, isomers, polymorphs, prodrugs and mixtures thereof, and also to its various crystalline and amorphous forms and any other derivatives or a mixture thereof. According to the present invention, brivaracetam is preferably present in the composition in an amount from about 5 to about 30% wt., more preferably from about 15 to about 20% wt.

The compound (2S)-2-[(4R)-2-oxo-4-propyltetrahydro-1H-pyrrol-1-yl]butanamide, also known as Brivaracetam, used herein has a particle size distribution such that more than 90% of the particles (d(0.9)) are between 10 μm to 100 μm. Preferably, more than 90% of the particles are between 35 μm to 60 μm and even more preferably, more than 90% of the particles are around 55 μm. The particle size distribution of the compound is measured using laser light diffraction (dry method) by Malvern Mastersizer 3000 or equivalent.

The term "binding agent" or "binding agent" as used herein is defined as an agent able to bind particles which cannot be bound only by a compression force. Examples of binding agents include starch, pregelatinized starch (PGS), cellulose, microcrystalline cellulose, silicified microcrystalline cellulose, methyl cellulose, ethyl cellulose, hydroxy propyl methyl cellulose (HPMC), hydroxypropyl cellulose (HPC), sodium carboxy methyl cellulose, polyvinyl pyrrolidone (PVP), polyethylene glycol (PEG), polyvinyl alcohols, polymethacrylates, polyvinylcaprolactam, vinylpyrrolidone-vinyl acetate

copolymers, and/or a mixtures or derivatives thereof.

The term "diluent" as used herein is defined as an inert agent designed to increase the weight and/or the size of the pharmaceutical composition, for example in the case of a tablet. Examples of diluents include, but are not limited to lactose anhydrous, lactose monohydrate, microcrystalline cellulose, mannitol and/or a mixture thereof. Preferred diluent according to the present invention is lactose monohydrate and/or microcrystalline cellulose and/or a mixture thereof. Each such diluent is present in an amount from about 30% wt. to about 40% wt. Preferably, the amount of diluent is about 35% wt. Most preferably, the diluent according to the present invention is a combination of anhydrous lactose and lactose monohydrate. Most preferably, the combination of anhydrous lactose and lactose monohydrate present is in an amount of about 70% wt.

The term "disintegrant" as used herein is defined as an accelerating agent of the disintegration of the tablet and the dispersion of the active ingredient in water or gastrointestinal fluids. Examples of disintegrants include, but are not limited to croscarmellose sodium, carboxymethyl cellulose calcium, crospovidone, potassium, sodium starch glycolate, starch, pregelatinized starch and/or a mixture thereof. Preferred disintegrant according to the present invention is croscarmellose sodium. Disintegrants are present in an amount from about 3 to about 10% wt. Preferably, the amount of disintegrant is from about 4% to about 9% wt. More preferably, the amount of disintegrant is from about 4% to about 7% wt. and most preferably, the amount of disintegrant is about 6% wt.

The term "lubricant" as used herein is defined as an agent able to decrease adhesion of a powder to punches and friction between particles. The lubricant may be present in the pharmaceutical composition in the form of a single compound or in the form of a mixture of compounds. Examples of lubricants include, but are not limited to talk, magnesium stearate, stearic acid, zinc stearate, sodium stearyl fumarate and/or a mixture thereof. Preferred lubricant according to the present invention is magnesium stearate. Lubricant is present in an amount from about 0.5% to about 2% wt., preferably, the amount of lubricant is from about 0.5% to 1.5% wt., most preferably, the amount of lubricant is about 1% wt.

The term "around" or "about" is used herein to mean approximately, roughly, or within a certain region of a specific value or a range, but not strictly and doctrinally limited to said value or a range, including the error margins as well. When the term "around" or "about" or similar terms as mentioned in this paragraph are used in conjunction with a numerical range or a value, it modifies that range by extending the boundaries both above and below the numerical values set by that range or a value. In general, this term is used herein to modify a numerical range or a value above and below the stated value by a variance of at least 10%.

The term "% wt." or "% by weight" is used herein to mean the percentage by weight of each ingredient in the uncoated or a coated tablet, including or excluding any coatings.

The term "Bulk Density" of a powder is defined is the ratio of the mass of an untapped powder sample and its volume including the contribution of the interparticulate void volume. It is usually expressed as g/ml and is obtained by measuring the volume of a fixed weight of powder after it has been tapped for a defined number of times.

The term "Tapped Density" is an increased bulk density attained after mechanically tapping a container containing the powder sample.

The term "Carr's Index" (CI) is used to predict the propensity (flowability) of a given powder sample to be compressed.

## Examples

The following examples are to illustrate some of the embodiments of the present invention without however limiting the scope of the invention, which is defined accordingly by the claims.

**Comparative example 1 - Formulation example with a cyclodextrin as a binding agent (Briviact - reference drug product), 100 mg strength**

| Material | Function | Formula (mg/tablet) | Ratio (%) |
|---|---|---|---|
| Brivaracetam | API | 100.00 | 18.52 |
| Lactose monohydrate | Diluent | 194.00 | 35.93 |
| Lactose anhydrous | Diluent | 193.00 | 35.74 |
| Croscarmellose sodium | Disintegrant | 20.00 | 3.70 |
| (2-hydroxypropyl)-β-cyclodextrin | Binding agent | 27.00 | 5.00 |
| Magnesium Stearate | Lubricant | 6.00 | 1.11 |
| **Core Tablet Weight** | | **540.00** | 100.0 |

(continued)

| Material | Function | Formula (mg/tablet) | Ratio (%) |
|---|---|---|---|
| Opadry II yellow-brown 85F270000 | | 16.20 | |
| **Total** | | **556.20** | |

The comparative example of a formulation with a cyclodextrin ((2-hydroxypropyl)-β-cyclodextrin) as a binding agent is the composition of the reference drug product Briviact, which is readily commercially available.

**Comparative example 2 - Formulation example with vinylpyrrolidone-vinyl acetate copolymer (Kollidon), 100 mg strength**

| Material | Function | Formula (mg/tablet) | Ratio (%) |
|---|---|---|---|
| Brivaracetam | API | 100.00 | 18.52 |
| Lactose monohydrate | Diluent | 187.60 | 34.74 |
| Lactose anhydrous | Diluent | 193.00 | 35.74 |
| Croscarmellose sodium | Disintegrant | 32.40 | 6.00 |
| Kollidon VA 64 | Binding agent | 21.60 | 4.00 |
| Magnesium Stearate | Lubricant | 5.40 | 1.00 |
| **Core Tablet Weight** | | **540.00** | 100.0 |
| Coating: Opadry II Tan 85F270000 | | 10.80 | |
| **Total** | | **550.80** | |

The formulation example with vinylpyrrolidone-vinyl acetate copolymer (Kollidon VA 64) as the single binding agent was manufactured according to the process specified below.

Manufacturing process:

1) Sifting and mixing: Sifting Brivaracetam, croscarmellose sodium, vinylpyrrolidone-vinyl acetate copolymer (Kollidon VA 64), lactose anhydrous, lactose monohydrate by sieving 0.6 mm and mixing it for 10 minutes;
2) Sifting and mixing: Adding ½ portion of magnesium stearate by sieving 0.25 mm to the mixture from step 1 and mixing it for 3 minutes;
3) Compaction: Dry granulation of the powder obtained in step 2 and sieving;
4) Mixing: After compaction, mixing of the obtained mixture for 5 minutes;
5) Sifting and mixing: Sieving the remaining portion of magnesium stearate by 0.25 mm and adding it to the mixture from step 4 and mixing it for 3 minutes.
6) Compression: Performing tablet compression;
7) Coating: Optionally, performing a coating process.

Brivaracetam composition prepared according to Example 2 has the following flow properties:

| Flow properties | |
|---|---|
| Carr's Index (CI) | 22 |
| Flow Properties | Passable |

**Comparative example 3 - Formulation example with hydroxypropyl cellulose (Klucel), 100 mg strength**

| Material | Function | Formula (mg/tablet) | Ratio (%) |
|---|---|---|---|
| Brivaracetam | API | 100.00 | 18.52 |
| Lactose monohydrate | Diluent | 187.60 | 34.74 |
| Lactose anhydrous | Diluent | 187.60 | 34.74 |
| Croscarmellose sodium | Disintegrant | 32.40 | 6.00 |
| Klucel | Binding agent | 27.00 | 5.00 |
| Magnesium Stearate | Lubricant | 5.40 | 1.00 |
| **Core Tablet Weight** | | **540.00** | 100.0 |
| Coating: Opadry II Tan 85F270000 | | 10.80 | |
| **Total** | | **550.80** | |

The formulation example with hydroxypropyl cellulose (Klucel) as the single binding agent was manufactured according to the process specified below.

Manufacturing process:

1) Sifting and mixing: Sifting Brivaracetam, croscarmellose sodium, hydroxypropyl cellulose (Klucel), lactose anhydrous, lactose monohydrate by sieving 0.6 mm and mixing it for 10 minutes;
2) Sifting and mixing: Adding ½ portion of magnesium stearate by sieving 0.25 mm to the mixture from step 1 and mixing it for 3 minutes;
3) Compaction: Dry granulation of the powder obtained in step 2 and sieving;
4) Mixing: After compaction, mixing of the obtained mixture for 5 minutes;
5) Sifting and mixing: Sieving the remaining portion of magnesium stearate by 0.25 mm and adding it to the mixture from step 4 and mixing it for 3 minutes.
6) Compression: Performing tablet compression;
7) Coating: Optionally, performing a coating process.

Brivaracetam composition prepared according to Example 3 has the following flow properties:

| **Flow properties** | |
|---|---|
| Carr's Index (CI) | 24 |
| Flow Properties | Passable |

**Formulation according to the invention**

**Example 4 - Formulation example without any binding agent, 100 mg strength**

| Material | Function | Formula (mg/tablet) | Ratio (%) |
|---|---|---|---|
| Brivaracetam | API | 100.00 | 18.52 |
| Lactose monohydrate | Diluent | 187.60 | 34.74 |
| Lactose anhydrous | Diluent | 214.60 | 39.74 |

(continued)

| Material | Function | Formula (mg/tablet) | Ratio (%) |
|---|---|---|---|
| Croscarmellose sodium | Disintegrant | 32.40 | 6.00 |
| Magnesium Stearate | Lubricant | 5.40 | 1.00 |
| **Core Tablet Weight** | | **540,00** | 100.0 |
| Coating: Opadry II Tan 85F270000 | | 10.80 | |
| **Total** | | **550.80** | |

The formulation example free of a cyclodextrin agent and free of a binding agent was manufactured according to the process specified below.

Manufacturing process:

1) Sifting and mixing: Sifting Brivaracetam, croscarmellose sodium, lactose anhydrous, lactose monohydrate by sieving 0.6 mm and mixing it for 10 minutes;
2) Sifting and mixing: Adding ½ portion of magnesium stearate by sieving 0.25 mm to the mixture from step 1 and mixing it for 3 minutes;
3) Compaction: Dry granulation of the powder obtained in step 2 and sieving;
4) Mixing: After compaction, mixing of the obtained mixture for 5 minutes;
5) Sifting and mixing: Sieving the remaining portion of magnesium stearate by 0.25 mm and adding it to the mixture from step 4 and mixing it for 3 minutes.
6) Compression: Performing tablet compression;
7) Coating: Optionally, performing a coating process.

Brivaracetam composition prepared according to Example 4 has the following flow properties:

| Flow properties | |
|---|---|
| Carr's Index (CI) | 20 |
| Flow Properties | Fair |

**Example 5 - Dissolution studies at 40°C stability for 2 months**

Dissolution studies were performed with three different formulations of Brivaracetam at 40°C stability for 2 months - formulation from example 2 (formulation with vinylpyrrolidone-vinyl acetate copolymer (Kollidon)), formulation from example 3 (formulation with hydroxypropyl cellulose (Klucel)) and formulation from example 4 (formulation example without any binding agent) and compared with the commercially available reference drug product Briviact (formulation as in comparative example 1), at conditions of pH 6.4 phosphate buffer, pedal 50 rpm for the tablets (100 mg).

| Time (min) | Briviact (example 1) 2 months / 40°C (%) | Example 2 2 months / 40°C (%) | Example 3 2 months / 40°C (%) | Example 4 2 months / 40°C (%) |
|---|---|---|---|---|
| **0** | 0.0 | 0.0 | 0.0 | 0.0 |
| **5** | 52 | 31 | 18 | 50 |
| **10** | 99 | 62 | 51 | 91 |
| **15** | 102 | 83 | 80 | 99 |
| **20** | 102 | 95 | 95 | 100 |
| **30** | 102 | 99 | 101 | 101 |
| **45** | 102 | 99 | 102 | 101 |

(continued)

| Time (min) | Briviact (example 1) 2 months / 40°C (%) | Example 2 2 months / 40°C (%) | Example 3 2 months / 40°C (%) | Example 4 2 months / 40°C (%) |
|---|---|---|---|---|
| **60** | 102 | 99 | 103 | 101 |

The results provided above show, that the dissolution results of the composition prepared according to Examples 2 and 3 are unexpectedly and significantly decreasing, especially at minutes 10 and 15 of the dissolution studies, and do not even reach an 85% dissolution of the composition at minute 15 of dissolution.

Surprisingly, the results also show that dissolution of the composition prepared according to Example 4 does not decrease at minutes 10 and 15 of dissolution studies and does reach more than 85% dissolution of the composition at minute 15 of dissolution and is also very similar and comparable with the reference drug product Briviact (Example 1) and is thus acceptable.

**Formulation according to the invention**

**Example 6 - Formulation examples without any binding agent in additional strengths**

| **Material** | **Function** | **Formula** | **Formula** | **Formula** | **Formula** |
|---|---|---|---|---|---|
| | | **(mg/tablet)** | **(mg/tablet)** | **(mg/tablet)** | **(mg/tablet)** |
| Brivaracetam | API | 10.00 | 25.00 | 50.00 | 75.00 |
| Lactose monohydrate | Diluent | 18.76 | 46.90 | 93.80 | 140.70 |
| Lactose anhydrous | Diluent | 21.46 | 53.65 | 107.30 | 160.95 |
| Croscarmellose sodium | Disintegrant | 3.24 | 8.10 | 16.20 | 24.30 |
| Magnesium Stearate | Lubricant | 0.54 | 1.35 | 2.70 | 4.05 |
| **Core Tablet Weight** | | **54.00** | **135.00** | **270.00** | **405.00** |
| Opadry II 85F18422 White | Coating | 1.08 | - | - | - |
| Opadry II 85F275014 Grey | | - | 2.70 | - | - |
| Opadry II 85F38197 Yellow | | - | - | 5.40 | - |
| Opadry II 85F200021 Purple | | - | | - | 8.10 |
| Opadry II 85F270000 Tan | | - | - | - | - |
| **Total** | | **55.08** | **137.70** | **275.40** | **413.10** |

The formulation examples in Example 6, free of a cyclodextrin agent and free of a binding agent, were prepared according to the same process described in Example 4. Note that the additional strengths are advantageously dose-proportional with 100 mg strength from Example 4, which further support the ease of preparation process of the formulation according to the present invention.

**Example 7** - Particle Size Distribution of Brivaracetam

The particle size distribution of Brivaracetam used in the formulation according to the invention was measured using laser light diffraction (dry method) by Malvern Mastersizer 3000. The obtained particle size distribution for such a sample were the following:

| | **Particle Size Distribution (μm)** |
|---|---|
| d (10) | 7.83 |
| d (50) | 20 |

(continued)

| | Particle Size Distribution (μm) |
|---|---|
| d (90) | 56.3 |

**List of used analytical methods**

The dissolution studies were performed according to FDA recommendation at conditions of pH 6.4 phosphate buffer with 900 ml volume, pedal apparatus with 50 rpm. Method details are given below.

Chromatographic Conditions:

| | |
|---|---|
| Instrument | : HPLC |
| Detector | : UV-VIS, PDA |
| Column | : Agilent Zorbax SB C18 (150 mm x 4.6 mm, 5 μm) |
| Wavelength | : 210 nm |
| Flow Rate | : 1.2 mL/dk |
| Injection Volume | : 5 μL |
| Analysis Time | : 8 min |
| Sample Temperature | : 25°C |
| Column Temperature | : 25°C |
| Buffer | : pH 6.4 |

Diluent: Mix acetonitrile and water in the ratio of 10:90 (% v/v).

Preparation of Buffer Solution: 1.24 grams of disodium hydrogen phosphate dihydrate, 2.83 grams of sodium dihydrogen phosphate and 8.2 grams of sodium chloride are weighed and dissolved in 950 ml of distilled water. Adjust the pH to 6.4 ± 0.05 with 1 M NaOH or 1 M HCl and make up to 1 liter.

Preparation of Mobile Phase:

| | |
|---|---|
| Mobile Phase A | : Add 1 mL of orthophosphoric acid to 1000 mL of water. |
| Mobile Phase B | : Acetonitrile |
| Mobile Phase | : Mix mobile phase-A and mobile phase-B in the ratio of 70:30 % v/v |

Dissolution Conditions:

| | |
|---|---|
| Apparatus | : Paddle |
| Dissolution Media | : pH:6.4 Buffer Solution |
| Speed | : 50 rpm |
| Dissolution Time | : 15 minutes |
| Temperature | : 370C ± 0.5 0C |
| Filter | : 70 μm Full-Flow Filter |
| Volume | : 900 mL |

Preparation of Stock Standard Solution: 11.0 mg Brivaracetam working standard is weighed into 50 mL volumetric flask and add to its half of the volume with diluent. Dissolved by keeping in ultrasonic bath for 15 minutes and shaked every 2-3 minutes. After ultrasonic bath, it is let to be reached room temperature and made up to its volume with dilüent. (CBrivaracetam= 0.22 mg/mL)

Preparation of Standard Solution for 10 mg: Transfer 5.0 mL of the Brivaracetam stock standard solution in a 100 mL volumetric flask and make up to volume with pH 6.4. This solution is filtered through selected filter and transferred to HPLC vial. (CBrivaracetam= 0.011 mg/mL)

Preparation of Standard Solution for 25 mg: Transfer 12.75 mL of the Brivaracetam stock standard solution in a 100 mL volumetric flask and make up to volume with pH 6.4. This solution is filtered through selected filter and transferred to HPLC vial. (CBrivaracetam= 0.028mg/mL)

Preparation of Standard Solution for 50 mg: Transfer 25.5 mL of the Brivaracetam stock standard solution in a 100 mL volumetric flask and make up to volume with pH 6.4. This solution is filtered through selected filter and transferred to HPLC vial. (CBrivaracetam= 0.056 mg/mL)

Preparation of Standard Solution for 75 mg: Transfer 3.75 mL of the Brivaracetam stock standard solution in a 10 mL volumetric flask and make up to volume with the solvent. This solution is filtered through selected filter and transferred to HPLC vial. (CBrivaracetam= 0.0825 mg/mL)

Preparation of Standard Solution for 100 mg: Transfer 5 mL of the Brivaracetam stock standard solution in a 10 mL volumetric flask and make up to volume with the solvent. This solution is filtered through selected filter and transferred to HPLC vial. (CBrivaracetam= 0.11 mg/mL)

Two control standard solutions are prepared identically with the standard solution.

Preparation of Sample Solution for 10 mg: 900 mL dissolution media is placed into each dissolution vessel. Dissolution media is brought to 37 °C ± 0.5 °C. Brivaracetam 10 mg tablet 6 tablets are weighed individually, and they are placed into the vessel at the same time. At the end of 15 minutes, samples are taken from each vessel by using 70 µm full flow filter into HPLC vial. (CBrivaracetam= 0. 011 mg/mL).

Preparation of Sample Solution for 25 mg: 900 mL dissolution media is placed into each dissolution vessel. Dissolution media is brought to 37 °C ± 0.5 °C. Brivaracetam 25 mg tablet 6 tablets are weighed individually, and they are placed into the vessel at the same time. At the end of 15 minutes, samples are taken from each vessel by using 70 µm full flow filter into HPLC vial. (CBrivaracetam= 0. 028 mg/mL).

Preparation of Sample Solution for 50 mg: 900 mL dissolution media is placed into each dissolution vessel. Dissolution media is brought to 37 °C ± 0.5 °C. Brivaracetam 50 mg tablet 6 tablets are weighed individually, and they are placed into the vessel at the same time. At the end of 15 minutes, samples are taken from each vessel by using 70 µm full flow filter into HPLC vial. (CBrivaracetam= 0. 055 mg/mL).

Preparation of Sample Solution for 75 mg: 900 mL dissolution media is placed into each dissolution vessel. Dissolution media is brought to 37 °C ± 0.5 °C. Brivaracetam 75 mg tablet 6 tablets are weighed individually, and they are placed into the vessel at the same time. At the end of 15 minutes, samples are taken from each vessel by using 70 µm full flow filter into HPLC vial. (CBrivaracetam= 0. 083 mg/mL).

Preparation of Sample Solution for 100 mg: 900 mL dissolution media is placed into each dissolution vessel. Dissolution media is brought to 37 °C ± 0.5 °C. Brivaracetam 100 mg tablet 6 tablets are weighed individually, and they are placed into the vessel at the same time. At the end of 15 minutes, samples are taken from each vessel by using 70 µm full flow filter into HPLC vial. (CBrivaracetam= 0.11 mg/mL).

System Suitability Test: Suitability of standard solution injections are controlled for following parameters. RSD between Brivaracetam peak areas obtained from 6 consecutive standard injections should be ≤ 2.0 %. The theoretical plate count of Brivaracetam peak obtained from standard preparation should be ≥ 2000. The symmetry factor of Brivaracetam peak obtained from standard preparation should be no more than 2.0.

Calculations:

Standard solution concentration is calculated by the following formula:

$$CStd = Wstd/V \times 5/10 \times P$$

Brivaracetam standard solution concentration (mg/mL)

| | |
|---|---|
| WStd | : Weigh of Brivaracetam working standard (mg) |
| V | : Dilution volume (mL) |
| P | : Potency of working standard (on as-is basis) |

Assay is calculated by the following formulas:

$$Brivaracetam\ (\%) = [A_Test/A_std] \times [C_std/(1/V)]\ x\ 100/(LC)$$

| | |
|---|---|
| ATest | : Peak area of Brivaracetam obtained to injection of test solution. |
| AStd | : Peak area of Brivaracetam obtained to injection of standard solution. |
| CStd | : Calculated Brivaracetam standard solution concentration (mg/mL) |
| V | : Dilution factor (900 mL) |
| LC | : Label claim |

Stability testing was performed according to ICH guideline Q1A(R2) (40°C stability for 2 months) Carr's Index (CI) was calculated based on the results of bulk and tapped density by using following formula:

$$CI = (\rho t - \rho b) / \rho t \ X \ 100$$

pt: Tapped density
pb: Bulk density

Expected flow properties (flowability) are categorized as follows:

| Carr's Index (CI) | Flowability expected |
|---|---|
| < 10 | Excellent / Very Free Flow |
| 11-15 | Good / Free Flow |
| 16-20 | Fair |
| 21 - 25 | Passable |
| 26 - 31 | Poor Flow / Cohesive |
| 32 - 37 | Very Poor Flow / Very Cohesive |
| > 38 | Approximatively no flow |

Particle Size Distribution (PSD) measurement using laser light diffraction (dry method) by Malvern Mastersizer 3000

The Operating Conditions of The Equipment to Be Used

| | |
|---|---|
| Instrument | : MALVERN MASTERSIZER 3000 |
| Mode | : Dry Method |
| Sensitivity | : Normal |
| Particle Refractive Index | : 1.615 |
| Absorption | : 0.01 |
| Measurement Size | : 6 second |
| Background Time | : 6 second |
| Air Pressure | : 2.5 ± 0.2 bar |
| Laser Obscuration | : Between 0.5% - 6.0% |
| Vibration feed rate | : 60% |
| Measurement Snaps | : 1200 |
| Background Snaps | : 1200 |

Preparation of Sample: Accurately weigh and transfer about 250mg of sample into a clean and dry sample feeder tray and measure the particle size distribution at d(0.1), d(0.5), d(0.9). Measure the particle size of the sample for 3 replicates for each measurement.

## Claims

1. A pharmaceutical composition comprising (2S)-2-[(4R)-2-oxo-4-propyltetrahydro-1H-pyrrol-1-yl]butanamide or its pharmaceutically acceptable salts, esters, solvates, polymorphs, enantiomers or mixtures thereof, which is free of

a cyclodextrin agent and free of a binding agent.

**2.** The pharmaceutical composition according to any of the preceding claims, further comprising excipients selected from a diluent, a disintegrant, a lubricant and/or a mixture thereof.

**3.** The pharmaceutical composition according to any of the preceding claims, comprising further excipients selected from either intra-granular excipients and/or extra-granular excipients.

**4.** The pharmaceutical composition according to claim 3, wherein the intra-granular excipients are selected from a diluent, a disintegrant, a binding agent and optionally a lubricant or at least part of the lubricant.

**5.** The pharmaceutical composition according to claim 3 and/or 4, wherein the extra-granular excipient is a lubricant or at least part of a lubricant.

**6.** The pharmaceutical composition according to any of the preceding claims, wherein the particle size distribution of (2S)-2-[(4R)-2-oxo-4-propyltetrahydro-1H-pyrrol-1-yl]butanamide or its pharmaceutically acceptable salts, esters, solvates, polymorphs, enantiomers or mixtures thereof is that more than d(0.9) is from 10 μm to 100 μm, preferably, d(0.9) is from 35 μm to 60 μm.

**7.** The pharmaceutical composition according to according any of the preceding claims, comprising:

a) a diluent which is lactose monohydrate and lactose anhydrous,
b) a disintegrant which is croscarmellose sodium,
c) a lubricant which is magnesium stearate, and
d) optionally a coating.

**8.** The pharmaceutical composition according to any of the preceding claims, comprising:

a) around 75% wt. of a diluent or a mixture of diluents,
b) around 6% wt. of a disintegrant,
c) around 1% wt. of a lubricant, and
d) optionally a coating layer of around 2% wt.

**9.** The pharmaceutical composition any of the preceding claims, comprising:

a) around 75% wt. of a diluent which is lactose monohydrate and lactose anhydrous
b) around 6% wt. of a disintegrant which is croscarmellose sodium,
c) around 1% wt. of a lubricant which is magnesium stearate, and
d) optionally a coating layer of around 2% wt.

**10.** A process for preparation of the pharmaceutical composition according to any of the claims 1 to 9, wherein the process is a dry granulation process.

**11.** The process according to claim 10, wherein the dry granulation process comprises a step of roller compactor of intra-granular excipients and a step of compression with extra-granular excipients.

**12.** The process according to claims 10 and/or 11, comprising the steps of sifting and mixing, compaction of intra-granular excipients, followed by sifting and mixing and compression with extra-granular excipients, optionally followed by a coating process.

**13.** The process according to any of the claims 10 to 12, comprising the steps of:

1) Sifting and mixing: Sifting Brivaracetam, croscarmellose sodium, lactose anhydrous, lactose monohydrate by sieving 0.6 mm and mixing it for 10 minutes;
2) Sifting and mixing: Adding ½ portion of magnesium stearate by sieving 0.25 mm to the mixture from step 1 and mixing it for 3 minutes;
3) Compaction: Dry granulation of the powder obtained in step 2 and sieving;
4) Mixing: After compaction, mixing of the obtained mixture for 5 minutes;

5) Sifting and mixing: Sieving the remaining portion of magnesium stearate by 0.25 mm and adding it to the mixture from step 4 and mixing it for 3 minutes.
6) Compression: Performing tablet compression;
7) Coating: Optionally, performing a coating process.

**14.** A pharmaceutical composition according to any of the claims 1 to 9, wherein at least 85% of the composition is dissolved within the first 15 minutes, at conditions of pH 6.4 phosphate buffer with 900 ml volume, pedal apparatus with 50 rpm.

**15.** A pharmaceutical composition according to any of the claims 1 to 9 and 14, obtained according to the process in any of the claims 10 to 13.

Europäisches Patentamt
European Patent Office
Office européen des brevets

# EUROPEAN SEARCH REPORT

Application Number
EP 23 16 2409

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 374 956 A (SHANGHAI DESANO BIOPHARMACEUTICALS CO LTD) 7 July 2020 (2020-07-07) * comparative examples 1-4, 7; claims 1-10; examples 1-18; tables * * paragraph [0098] * ----- | 1-15 | INV. A61K9/20 A61K31/4015 |
| X | CN 113 876 726 A (NINGBO HIGH NEW DISTRICT MENOVO PHARMACEUTICAL INNOVATION RES INSTITUT) 4 January 2022 (2022-01-04) * abstract; claims 1-9; figure 1; examples 1-5 * ----- | 1-15 | |
| X | CN 110 638 743 A (LEPU PHARMACEUTICAL TECH CO LTD) 3 January 2020 (2020-01-03) * Formulations 1-9; examples 1-5 * ----- | 1-6,8, 10-15 | |
| X | CN 112 569 198 A (ZHEJIANG HEZE PHARMACEUTICAL TECH CO LTD ET AL.) 30 March 2021 (2021-03-30) * claims 1-7; examples 1-5 * ----- | 1-5,8, 10-12, 14,15 | TECHNICAL FIELDS SEARCHED (IPC) |
| X | WO 2017/195144 A1 (JUBILANT GENERICS LTD [IN]) 16 November 2017 (2017-11-16) * claims 1-10; examples 1-4; tables 1-2 * ----- | 1-6, 10-12, 14,15 | A61K |

The present search report has been drawn up for all claims

1

| Place of search | Date of completion of the search | Examiner |
|---|---|---|
| Munich | 2 August 2023 | Madalinska, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 16 2409

This annex lists the patent family members relating to the patent documents cited in the above-mentioned European search report. The members are as contained in the European Patent Office EDP file on
The European Patent Office is in no way liable for these particulars which are merely given for the purpose of information.

02-08-2023

| Patent document cited in search report | | Publication date | Patent family member(s) | | Publication date |
|---|---|---|---|---|---|
| CN 111374956 | A | 07-07-2020 | NONE | | |
| CN 113876726 | A | 04-01-2022 | NONE | | |
| CN 110638743 | A | 03-01-2020 | NONE | | |
| CN 112569198 | A | 30-03-2021 | NONE | | |
| WO 2017195144 | A1 | 16-11-2017 | US 2019125725 | A1 | 02-05-2019 |
| | | | WO 2017195144 | A1 | 16-11-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0162726 A
- WO 0164637 A
- WO 2010094535 A
- WO 2010086315 A